# EUROPÄISCHE PATENTSCHRIFT

(11) **EP 0 377 150 B1**
(45) Veröffentlichungstag und Bekanntmachung des Hinweises auf die Patenterteilung: **09.03.1994**
(21) Anmeldenummer: 89123115.1
(22) Anmeldetag: 14.12.1989
(51) Int. Cl.: C07C 43/13, C07C 41/03, C07C 41/26

(54) **Verfahren zur Herstellung von Polyglycerinen**
Process for the preparation of polyglycerines
Procédé pour la préparation de polyglycérines

(30) Priorität: 03.01.1989 DE 3900059
(43) Veröffentlichungstag der Anmeldung: 11.07.1990
(73) Patentinhaber: DEUTSCHE SOLVAY-WERKE GMBH, 42697 Solingen (DE)
(72) Erfinder: Jakobson, Gerald, Dr. Dipl.-Chem., D-4134 Rheinberg 2 (DE); Siemanowski, Werner, Dr. Dipl.-Chem., D-4134 Rheinberg 1 (DE)
(74) Vertreter: Lauer, Dieter, Dr.

(56) Entgegenhaltungen:
- DE-A- 3 410 520
- FR-A- 1 055 569
- US-A- 2 520 670
- US-A- 4 053 525

## Beschreibung

Die vorliegende Erfindung betrifft ein Verfahren zur Herstellung von Polyglycerinen (mit mehr als 50 Gew.-% an Diglycerin), die arm an cyclischen Komponenten sind, durch Umsetzung von Chlorhydrinen.

Aus der FR-A-1055569 ist es bereits bekannt, Epichlorhydrin in Gegenwart eines Katalysators auf Chlorhydrine des Glykols, die eine freie OH-Gruppe aufweisen, einwirken zu lassen, um polychlorierte Ether oder polychlorierte Polyether zu erhalten.

Gemäß US-A-4053525 wird Glycerin aus Chlorhydrin und Epichlorhydrin enthaltenen Gemischen in Gegenwart eines Alkalimetallcarbonates unter Mitverwendung einer organischen Lösemittelphase, die weniger als etwa 0,5 Gew.-% Löslichkeit in Wasser aufweist und eine Dielektrizitätskonstante von weniger als 10 besitzt, hergestellt. Dabei wird somit ein organisches, wasserschwerlösliches Lösemittel eingesetzt und die Wasser-Ölphase für einen Zeitraum von 20 bis 150 Stunden zur Reaktion gebracht. Die verwendeten organisch-chemischen Lösemittel sind z.B. aromatische Kohlenwasserstoffe wie Xylol oder Toluol, aliphatische, chlorierte Kohlenwasserstoffe und dergleichen.

Gemäß US-PS 4053525 wird das organisch-chemische Lösemittel zusätzlich im Kreislauf geführt, so daß nach z.B. 44 Stunden 8,9 % Glycerin und nach 90 Stunden 10,3 % Glycerin entstanden sind. Dieses Verfahren ist daher großtechnisch überhaupt nicht anwendbar und praktizierbar.

Ferner muß berücksichtigt werden, daß es sich bei Polyglycerinen um chemisch andere und anders reagierende Verbindungen handelt als bei Glycerin selbst. Di-, Tri-, Tetra- und andere Polyglycerine besitzen im Gegensatz zu Monoglycerin Oxy-Etherverbindungen, die weder in ihrer chemischen Reaktion noch in ihren sonstigen Eigenschaften vergleichbar sind mit Glycerin.

Bei dieser in Gegenwart organisch-chemischer Lösemittel durchgeführten Reaktion, wird Glycerin hergestellt, das ein Minimum von Glycerinetherderivaten (also Diglycerin) und ähnlichen Verbindungen enthält.

Aus der US-PS 2520670 ist ein Verfahren zur Herstellung von Polyglycerinen bekannt, bei dem Glycerin-α-Monochlorhydrin mit Glycerin in Gegenwart von konzentriertem Alkali bei erhöhter Temperatur zu einem Gemisch von Polyglycerinen umgesetzt wird. Dieses Verfahren hat den Nachteil der relativ langen Reaktionsdauer, des hohen Anteils an Polyglycerin und, daß nach Beendigung der Reaktion das Reaktionsgemisch mit niederen aliphatischen Alkoholen aufgearbeitet werden muß.

Angaben über die erzielten Ausbeuten an Polyglycerinen sowie über deren Gehalt an cyclischen Komponenten werden nicht gemacht.

Bei der Patentanmeldung DE-A-3410520 wird von einem Destillationsrückstand aus der synthetischen Glycerinherstellung über Epichlorhydrin ausgegangen. Bei diesem Verfahren werden als Hauptprodukt nicht Polyglycerin, sondern das Glycerin (Monoglycerin) hergestellt und es wird auch kein α-Monochlorhydrin mit Epichlorhydrin umgesetzt.

Der bei der Destillation des Monoglycerins als Hauptprodukt anfallende Destillationsrückstand stellt eine zähe, bei Umgebungstemperatur feste bis breiige Masse mit einem sehr hohen Salzgehalt dar und wird volkstümlich wegen seiner Zähigkeit und dunklen Verfärbung auch Glycerinpech genannt. Dieser Destillationsrückstand aus der Monoglycerinherstellung ist in keiner Weise, weder hinsichtlich seiner Zusammensetzung noch seines physikalischen Zustandes, in der Art der Herstellung mit dem anfallenden Reaktionsgemisch gemäß US-A-2520670 vergleichbar.

Aufgabe der vorliegenden Erfindung war es daher, ein Verfahren zu finden, mit dessen Hilfe Polyglycerine in guten Ausbeuten und mit nur geringem Anteil an cyclischen Komponenten erhalten werden. Hierbei sollte gleichzeitig eine Isolierung der Zwischenprodukte (Chlorhydrinethermischung) nicht erforderlich sein sowie eine Aufarbeitung der Endprodukte durch Behandlung mit organischen Lösungsmitteln vermieden werden.

Erfindungsgemäß wurde festgestellt, daß diese Aufgabe dadurch gelöst wird, daß Epichlorhydrin mit α-Monochlorhydrin bei Temperaturen von 20 bis 120°C, vorzugsweise 50°C bis 100°C, in einem Molverhältnis von Epichlorhydrin zu α-Monochlorhydrin von 0,8 : 1 bis 1 : 2,5 in Gegenwart von Säuren oder sauer reagierenden Verbindungen umgesetzt, wobei nach der Umsetzung von Epichlorhydrin mit α-Monochlorhydrin nicht umgesetztes und/oder im Überschuß eingesetztes α-Monochlorhydrin und/oder Epichlorhydrin durch Destillation entfernt sowie in den Prozeß zurückgeführt wird, und dem erhaltenen, nicht aufgetrennten Reaktionsgemisch bei einer Temperatur von 50°C bis 120°C, vorzugsweise 80°C bis 95°C, dem Gehalt des Reaktionsgemisches an organisch gebundenem Chlor ein alkalisch reagierendes Medium, vorzugsweise eine alkalisch reagierende wäßrige Lösung im äquivalenten Verhältnis von Alkalicarbonat zu dem Gehalt an organisch gebundenem Chlor von 1,05 : 1 bis 1,1 : 1, zugegeben und das in der alkalischen Lösung umgesetzte Reaktionsgemisch durch Wasserzugabe auf eine 70 - 40 gew.-%ige Lösung verdünnt sowie bei Temperaturen von 30°C bis 80°C über eine Kombination von stark sauren Kationen- und nachfolgend schwach basischen Anionenaustauschern, die temperaturbeständig bis über 80°C sind und eine innere Oberfläche (gemessen nach Methode BET) von mehr als 25 m²/g aufweisen, entsalzt wird, durch Destillation entwässert und das Diglycerin-Polyglyceringemisch (mit einem Anteil an Glycerin), das arm an cyclischen Komponenten ist, durch fraktionierte Destillation in Diglycerin, höhere Polyglycerine und ggf. Glycerin aufgetrennt wird.

Als Säure oder sauer reagierende Verbindungen werden im erfindungsgemäßen Verfahren Mineralsäuren, Lewissäuren, halogenierte Carbonsäure, vorzugsweise Schwefelsäure, Phosphorsäure oder phosphorige Säure, Bortrifluorid, Bortrifluoretherat, Eisen-III-Chlorid und/oder Zinktetrachlorid, Trifluoressigsäure und/oder Chloressigsäure, in einer Konzentration von 0,1 bis 2 Gew.-%, vorzugsweise von 0,1 bis 1 Gew.-%, bezogen auf die Gewichtsmenge des eingesetzten α-Monochlorhydrin, eingesetzt.

Nach einer vorteilhaften Ausführungsform der Erfindung wird das in einer alkalischen Lösung umgesetzte Reaktionsgemisch durch Wasserzugabe auf eine etwa 60 - 50 gew.- %ige Lösung verdünnt und bei Temperaturen vorzugsweise von 40 °C bis 60 °C über eine Kombination aus stark sauren Kationen- und nachfolgenden schwach basischen Anionenaustauschern entsalzt.

Nach einer bevorzugten Ausführungsform wird dem nicht aufgetrennten Reaktionsgemisch aus der Umsetzung von Epichlorhydrin mit α-Monochlorhydrin oder einem höheren Polyglycerin eine alkalisch reagierende Alkalicarbonatlösung, vorzugsweise eine konzentrierte Sodalösung, zugegeben.

Zweckmäßig wird das nicht aufgetrennte Reaktionsgemisch aus der Umsetzung von Epichlorhydrin mit α-Monochlorhydrin, das Diglycerin und ggf. ihre höhere Polyglycerine enthält, mit der alkalisch reagieren-den Lösung, vorzugsweise Sodalösung, unter Zwangsbewegung vermischt, wobei vorzugsweise das nicht aufgetrennte Reaktionsgemisch aus der Umsetzung von Epichlorhydrin mit α-Monochlorhydrin zu der vorgelegten alkalisch reagierenden Lösung, vorzugsweise Sodalösung, zugegeben wird.

Vorteilhaft wird somit dem Umsetzungsprodukt von Epichlorhydrin mit α-Monochlorhydrin eine alkalisch reagierende Lösung, vorzugsweise Sodalösung in dem äquivalenten Verhältnis von Alkalicarbonat zu dem Gehalt an organisch-gebundenem Chlor von vorzugsweise 1,05 : 1 bis 1,1 : 1, zugegeben.

Nach einer weiteren Ausführungsform wird nach der Umsetzung von Epichlorhydrin mit α-Monochlorhydrin nicht umgesetztes und/oder im Überschuß eingesetztes α-Monochlorhydrin und/oder Epichlorhydrin durch Destillation, vorzugsweise Vakuum-Destillation, entfernt sowie in den Prozeß zurückgeführt und nachfolgend die alkalische Hydrolyse durchgeführt.

Zweckmäßig weist das Gemisch aus dem Reaktionsgemisch und der alkalisch reagierenden wäßrigen Lösung einen pH-Bereich von 7,0 bis 13, vorzugsweise 8 bis 12, auf.

Nach einer weiteren Ausführungsform wird das Reaktionsgemisch nach der Umsetzung auf Raumtemperatur abgekühlt und die Hauptmenge des ausgefällten Salzes abgetrennt, vorzugsweise abfiltriert.

Die Regenerierung der Kationenaustauschermasse in den Kationenaustauschern erfolgt mittels einer Gleichstrom- oder Verbund-Gleichstrom-Regenerierung.

Die Durchleitung der diglycerinhaltigen Lösung durch die Ionenaustauscher erfolgt vorteilhaft unter Überdruck.

Vorzugsweise wird die diglycerinhaltige Lösung unter einem Druck von 1,1 bis 10 bar, vorzugsweise 2 bis 6 bar, durch einen oder mehrere Kationenaustauscher und mindestens einen Anionenaustauscher geleitet.

Zweckmäßig ist die Ionenaustauschermasse des Kationenaustauschers und/oder Anionenaustauschers von einer Siebplatte, Lochplatte oder einer in der Höhenrichtung des Ionenaustauschers verschiebbar angeordneten, die Austauschermasse abdeckenden und einen gleichmäßigen Flüssigkeitsdurchtritt ermöglichenden Vorrichtung und/oder einer inerten Preßmasse und/ oder elastischen Kunststoffmasse bedeckt.

Die eingesetzten Kationenaustauschermassen und Anionenaustauschermassen sind vorteilhaft temperaturbeständig bis über 80 °C, vorzugsweise bis über 100 °C.

Die stark sauere Kationenaustauschermasse und die schwach basische Anionenaustauschermasse weisen bevorzugt eine innere Oberfläche (gemessen nach Methode BET) von mehr als 25/m², vorzugsweise 50 bis 100/m² auf.

Die diglycerinhaltige Lösung wird vorzugsweise mit einer Strömungsgeschwindigkeit von 0,5 m/h bis 15 m/h, vorzugsweise 1 m/h bis 5 m/h, durch die Ionenaustauscher geleitet.

### Ausführungsbeispiele:

1. 1,326 kg (12 mol) α-Monochlorhydrin und 2 ml SnCl₄ werden in einen 2-1-Doppelwandreaktor (Heizflüssigkeit: Öl; Inertgasatmosphäre: N gegeben und auf ca. 60 °C erwärmt.
   Innerhalb von 2 h werden 1,11 kg (12 mol) Epichlorhydrin so schnell zugetropft, daß 80 °C nicht überschritten werden (gegebenenfalls wird das Thermoöl über einen Wärmeaustauscher abgekühlt). Nach einer weiteren Stunde bei 70 °C ist die Reaktion beendet.
   Die dem Gehalt an organisch gebundenem Chlor der oben dargestellten Reaktionslösung entsprechende Menge (plus 10 % Überschuß) einer 2 molaren Sodalösung wird auf ca. 90 °C erhitzt. Unter Rühren wird die rohe Chlorhydrinethermischung innerhalb von 2 h zudosiert. Nach einer weiteren Stunde bei 90 °C wird die Heizung abgestellt und der Reaktionsansatz durch Zugabe 1/2 konzentrierter Salzsäure neutralisiert.
   Die neutrale Reaktionslösung wird im Vakuum eingeengt, die ausgefallenen Salze abfiltriert und das Filtrat nach Verdünnung mit Wasser über eine Kombination von Kationen- und Anionenaustauschern entsalzt. Zur Entwässerung wird diese Roh-Polyglycerinlösung im Vakuum eingedampft.
   Das Produktgemisch hatte folgende Zusammensetzung (in Gew.-%):
   Glycerin 44,3, cyclisches Diglycerin 0,4, Diglycerin 38,7, cyclisches Triglycerin 0,3, Triglycerin 12,7, cyclisches Tetraglycerin 0,3, Tetraglycerin 2,8, Pentaglycerin 0,5.
2. 1,326 kg (12 mol) α-Monochlorhydrin und 8 ml phosphorige Säure (30%ig in Wasser) werden in einem 2-1-Doppelwandreaktor (Heizflüssigkeit : Öl) gegeben und auf 80 °C erwärmt.
   Innerhalb von 2 h werden 1,11 kg (12 mol) Epichlorhydrin so schnell zugetropft, daß 95 °C nicht überschritten werden (gegebenenfalls wird das Thermoöl über einen Wärmetauscher abgekühlt). Nach einer weiteren Stunde bei 85 °C ist die Reaktion beendet.
   Die Hydrolyse der Reaktionslösung sowie die Aufarbeitung erfolgen wie im Beispiel 1 beschrieben.
   Das Produktgemisch hatte folgende Zusammensetzung (in Gew.-%):
   Glycerin 46,1, cyclisches Diglycerin 0,6, Diglycerin 40,8, cyclisches Triglycerin 0,6, Triglycerin 9,2, cyclisches Tetraglycerin 0,3, Tetraglycerin 2,1, Pentaglycerin 0,3.

## Patentansprüche

1. Verfahren zur Herstellung von Polyglycerinen (mit mehr als 50 Gew.-% Diglycerin) durch Umsetzung von Chlorhydrinen, dadurch gekennzeichnet, daß Epichlorhydrin mit α-Monochlorhydrin bei Temperaturen von 20 bis 120 °C in einem Molverhältnis von Epichlorhydrin zu α-Monochlorhydrin von 0,8 : 1 bis 1 : 2,5 in Gegenwart von Säuren oder sauer reagierenden Verbindungen umgesetzt, wobei nach der Umsetzung von Epichlorhydrin mit α-Monochlorhydrin nicht umgesetztes und/oder im Überschuß eingesetztes α-Monochlorhydrin und/oder Epichlorhydrin durch Destillation entfernt sowie in den Prozeß zurückgeführt wird, und dem erhaltenen, nicht aufgetrennten Reaktionsgemisch bei einer Temperatur von 50 °C bis 120°C, entsprechend dem Gehalt des Reaktionsgemisches an organisch gebundenem Chlor ein alkalisch reagierendes Medium, vorzugsweise eine alkalisch reagierende wäßrige Lösung im äquivalenten Verhältnis von Alkalicarbonat zu dem Gehalt an organisch gebundenem Chlor von 1,05 : 1 bis 1,1 : 1, zugegeben und das in der alkalischen Lösung umgesetzte Reaktionsgemisch durch Wasserzugabe auf eine 70 - 40 gew.-%ige Lösung verdünnt sowie bei Temperaturen von 30 °C bis 80 °C über eine Kombination von stark sauren Kationen- und nachfolgend schwach basischen Anionenaustauschern, die temperaturbeständig bis über 80 °C sind und eine innere Oberfläche (gemessen nach Methode BET) von mehr als 25 m²/g aufweisen, entsalzt wird, durch Destillation entwässert und das Diglycerin-Polyglyceringemisch (mit einem Anteil an Glycerin), das arm an cyclischen Komponenten ist, durch fraktionierte Destillation in Diglycerin, höhere Polyglycerine und ggf. Glycerin aufgetrennt wird.

2. Verfahren nach Anspruch 1, dadurch gekennzeichnet, daß als Säuren oder sauer reagierende Verbindungen Mineralsäuren, Lewissäuren und/oder halogenierte Carbonsäuren, vorzugsweise Schwefelsäure, Phosphorsäure oder phosphorige Säure, Bortrifluorid, Bortrifluoretherat, Eisen-III-Chlorid und/oder Zinktetrachlorid, Trifluoressigsäure und/oder Chloressigsäure, in einer Konzentration von 0,1 bis 2 Gew.-%, vorzugsweise von 0,1 bis 1 Gew.-%, bezogen auf die Gewichtsmenge des eingesetzten α-Monochlorhydrin, eingesetzt werden.

3. Verfahren nach Ansprüchen 1 oder 2, dadurch gekennzeichnet, daß das in der alkalischen Lösung umgesetzte Reaktionsgemisch durch Wasserzugabe auf eine 60 - 50 gew.%-ige Lösung verdünnt sowie bei Temperaturen von 40 °C bis 60 °C über die Kombination von stark sauren Kationen- und nachfolgenden schwach basischen Anionenaustauschern entsalzt wird.

4. Verfahren nach einem oder mehreren der Ansprüche 1 bis 3, dadurch gekennzeichnet, daß dem nicht aufgetrennten Reaktionsgemisch aus der Umsetzung von Epichlorhydrin mit α-Monochlorhydrin oder einem höheren Polyglycerin eine alkalisch reagierende Alkalicarbonatlösung, vorzugsweise eine konzentrierte Sodalösung, zugegeben wird.

5. Verfahren nach einem oder mehreren der Ansprüche 1 bis 4, dadurch gekennzeichnet, daß das nicht aufgetrennte Reaktionsgemisch aus der Umsetzung von Epichlorhydrin mit α-Monochlorhydrin, das Diglycerin und ggf. höhere Polyglycerine enthält, mit der Sodalösung, unter Zwangsbewegung vermischt wird, wobei das nicht aufgetrennte Reaktionsgemisch aus der Umsetzung von Epichlorhydrin mit α-Monochlorhydrin zu der vorgelegten Sodalösung zugegeben wird.

6. Verfahren nach einem oder mehreren der Ansprüche 1 bis 5, dadurch gekennzeichnet, daß das Gemisch aus dem Reaktionsgemisch und der alkalisch reagierenden wäßrigen Lösung einen pH-Bereich von
7,0 bis 13, vorzugsweise
8 bis 12,
aufweist.

7. Verfahren nach einem oder mehreren der Ansprüche 1 bis 6, dadurch gekennzeichnet, daß das Reaktionsgemisch nach der Umsetzung auf Raumtemperatur abgekühlt und die Hauptmenge des ausgefällten Salzes abgetrennt, vorzugsweise abfiltriert wird.

8. Verfahren nach einem oder mehreren der Ansprüche 1 bis 7, dadurch gekennzeichnet, daß die Regenerierung der Kationenaustauschermasse in den Kationenaustauschern mittels einer Gleichstrom- oder Verbund-Gleichstrom-Regenerierung erfolgt.

9. Verfahren nach einem oder mehreren der Ansprüche 1 bis 8, dadurch gekennzeichnet, daß die Durchleitung der diglycerinhaltigen Lösung durch die Ionenaustauscher unter Überdruck erfolgt.

10. Verfahren nach einem oder mehreren der Ansprüche 1 bis 10, dadurch gekennzeichnet, daß die diglycerinhaltige Lösung unter einem Druck von
1,1 - 10 bar, vorzugsweise
2 - 6 bar,
durch einen oder mehrere Kationenaustauscher und mindestens einen Anionenaustauscher geleitet wird.

11. Verfahren nach einem oder mehreren der Ansprüche 1 bis 10, dadurch gekennzeichnet, daß die Ionenaustauschermasse des Kationenaustauschers und/oder Anionenaustauschers von einer Siebplatte, Lochplatte oder einer in der Höhenrichtung des Ionenaustauschers verschiebbar angeordneten, die Austauschermasse abdeckenden und einen gleichmäßigen Flüssigkeitsdurchtritt ermöglichenden Vorrichtung und/oder einer inerten Preßmasse und/oder elastischen Kunststoffmasse bedeckt ist.

12. Verfahren nach einem oder mehreren der Ansprüche 1 bis 11, dadurch gekennzeichnet, daß die eingesetzten Kationenaustauschermassen und Anionenaustauschermassen temperaturbeständig
bis über 100 °C,
sind.

13. Verfahren nach einem oder mehreren der Ansprüche 1 bis 12, dadurch gekennzeichnet, daß die stark saure Kationenaustauschermasse und die schwach basische Anionenaustauschermasse eine innere Oberfläche (gemessen nach Methode BET) von
50 bis 100 m²/g,
aufweisen.

14. Verfahren nach einem oder mehreren der Ansprüche 1 bis 13, dadurch gekennzeichnet, daß die diglycerinhaltige Lösung mit einer Strömungsgeschwindigkeit von
0,5 m/h bis 15 m/h, vorzugsweise
1 m/h bis 5 m/h,
durch die Ionenaustauscher geleitet wird.

## Claims

1. A method for preparing polyglycerols (containing more than 50% by weight diglycerol) by reacting chlorohydrins, characterised in that epichlorohydrin is reacted with α-monochlorohydrin at temperatures of 20 to 120°C in a molar ratio of epichlorohydrin to α-monochlorohydrin of 0.8 : 1 to 1 : 2.5 in the presence of acids or acid-reacting compounds, wherein after the reaction of epichlorohydrin with α-monochlorohydrin α-monochlorohydrin and/or epichlorohydrin which has not been reacted and/or which has been used in an excess is removed by distillation and is recycled into the process, and an alkaline-reacting medium, preferably an alkaline-reacting aqueous solution in an equivalent ratio of alkali carbonate to the content of organically bound chlorine of 1.05 : 1 to 1.1 : 1 is added to the resulting, non-separated reaction mixture at a temperature of 50°C to 120°C, corresponding to the content of organically bound chlorine in the reaction mixture, and the reaction mixture reacted in the alkaline solution is diluted to a 70 - 40% by weight solution by the addition of water and is desalted at temperatures of 30°C to 80°C by means of a combination of strongly acidic cation exchangers and subsequently weakly basic anion exchangers which are temperature-resistant to above 80°C and have an internal surface area (measured according to the BET method) of more than 25 m²/g, is dehydrated by distillation and the diglycerol-polyglycerol mixture (with a content of glycerol), which is low in cyclic components, is separated into diglycerol, higher polyglycerols and optionally glycerol by fractional distillation.

2. A method according to Claim 1, characterised in that mineral acids, Lewis acids and/or halogenated carboxylic acids, preferably sulphuric acid, phosphoric acid or phosphorous acid, boron trifluoride, boron trifluoroetherate, iron III chloride and/or zinc tetrachloride, trifluoroacetic acid and/or chloracetic acid, in a concentration of 0.1 to 2% by weight, preferably of 0.1 to 1% by weight, relative to the quantity by weight of the α-monochlorohydrin used, are used as acids or acid-reacting compounds.

3. A method according to Claims 1 or 2, characterised in that the reaction mixture reacted in the alkaline solution is diluted by the addition of water to form a 60 - 50% by weight solution and is desalted at temperatures of 40°C to 60°C, by means of the combination of strongly acidic cation exchangers and subsequent weakly basic anion exchanger.

4. A method according to one or more of Claims 1 to 3, characterised in that an alkaline-reacting alkali carbonate solution, preferably a concentrated soda solution, is added to the non-separated reaction mixture from the reaction of epichlorohydrin with α-monochlorohydrin or a higher polyglycerol.

5. A method according to one or more of Claims 1 to 4, characterised in that the non-separated reaction mixture from the reaction of epichlorohydrin with α-monochlorohydrin, which contains diglycerol and optionally higher polyglycerols, is mixed with the soda solution with forced movement, the non-separated reaction mixture from the reaction of epichlorohydrin with α-monochlorohydrin being added to the soda solution which has been introduced.

6. A method according to one or more of Claims 1 to 5, characterised in that the mixture of the reaction mixture and the alkaline-reacting aqueous solution has a pH range of
7.0 to 13, preferably
8 to 12.

7. A method according to one or more of Claims 1 to 6, characterised in that the reaction mixture after the reaction is cooled to room temperature and the greater part of the precipitated salt is separated off, preferably filtered off.

8. A method according to one or more of Claims 1 to 7, characterised in that the regeneration of the cation exchanger substance in the cation exchangers takes place by means of co-current or compounded-co-current regeneration.

9. A method according to one or more of Claims 1 to 8, characterised in that the diglycerol-containing solution is passed through the ion exchangers under excess pressure.

10. A method according to one or more of Claims 1 to 9, characterised in that the diglycerol-containing solution is passed through one or more cation exchangers and at least one anion exchanger at a pressure of
1.1 - 10 bar, preferably
2 - 6 bar.

11. A method according to one or more of Claims 1 to 10, characterised in that the ion exchanger substance of the cation exchanger and/or anion exchanger is covered by a sieve plate, perforated plate or a device which is arranged to be displaceable in the vertical direction of the ion exchanger, covers the exchanger substance and permits even penetration by liquid, and/or by an inert moulded compound and/or elastic plastic substance.

12. A method according to one or more of Claims 1 to 11, characterised in that the cation exchanger substances and anion exchanger substances used are heat-resistant to
above 100°C.

13. A method according to one or more of Claims 1 to 12, characterised in that the strongly acidic cation exchanger substance and the weakly basic anion exchanger substance have an internal surface area (measured according to the BET method) of
50 to 100 m²/g.

14. A method according to one or more of Claims 1 to 13, characterised in that the diglycerol-containing solution is passed through the ion exchangers at a flow rate of
0.5 m/h to 15 m/h, preferably
1 m/h to 5 m/h.

## Revendications

1. Procédé de préparation de polyglycérols (comportant plus de 50 % en poids de diglycérol) par la réaction dechlorohydrines, caractérisé en ce qu'on fait réagir l'épichlorohydrine avec l'α-monochlorohydrine à des températures de 20 à 120°C, selon un rapport molaire de l'épichlorohydrine à l'α-monochlorohydrine de 0,8:1 à 1:2,5 en présence d'acides ou de composés à réaction acide, et, après la réaction de l'épichlorohydrine avec l'α-monochlorohydrine, on sépare par distillation l'α-monochlorohydrine et/ou l'épichlorohydrine n'ayant pas réagi et/ou présente en excès, et on la ou les recycle dans le procédé, et l'on ajoute au mélange réactionnel obtenu, n'ayant pas subi de séparation de ses constituants, à une température de 50°C à 120°C, selon la teneur du mélange réactionnel en chlore organiquement lié, un milieu à réaction alcaline, avantageusement une solution aqueuse à réaction alcaline, introduite selon un rapport entre les équivalents de carbonate alcalin et la teneur en chlorure organiquement lié de 1,05:1 à 1,1:1, et l'on dilue le mélange réactionnel ayant réagi dans la solution alcaline en lui ajoutant de l'eau jusqu'à obtenir une solution à 70-40 % en poids que l'on dessale à des températures de 30°C à 80°C sur une combinaison d'échangeurs de cations fortement acides, puis d'échangeurs d'anions faiblement basiques, qui sont stables à des températures allant jusqu'à plus de 80°C et présentent une surface spécifique intérieure (mesurée selon la méthode BET) de plus de 25 m²/g, on élimine l'eau par distillation et l'on soumet le mélange diglycérol-polyglycérols (présentant une certaine proportion de glycérol) et qui est pauvre en des constituants cycliques, à une séparation par distillation fractionnée donnant de la diglycérine, des polyglycérines supérieurs et éventuellement du glycérol.

2. Procédé selon la revendication 1, caractérisé en ce qu'on utilise comme acides ou composés à réaction acide des acides minéraux, des acides de Lewis et/ou des acides carboxyliques halogénés, avantageusement l'acide sulfurique, l'acide phosphorique, ou l'acide phosphoreux, le trifluorure de bore, le complexe éthéré de trifluorure de bore, le chlorure de fer-III et/ou le tétrachlorure de zinc, l'acide trifluoroacétique et/ou l'acide chloroacétique, en une concentration de 0,1 à 2 % en poids, avantageusement de 0,1 a 1 % en poids, par rapport à la quantité pondérale de l'α-monochlorohydrine mise en oeuvre.

3. Procédé selon les revendications 1 ou 2, caractérisé en ce qu'on dilue le mélange réactionnel, ayant réagi dans la solution alcaline, en lui ajoutant de l'eau jusqu'à obtenir une solution à 60-50 % en poids et qu'on le dessale à des températures de 40°C à 60°C sur la combinaison d'échangeurs de cations fortement acides, puis d'échangeurs d'anions faiblement basiques.

4. Procédé selon une ou plusieurs des revendications 1 à 3, caractérisé en ce qu'on ajoute une solution de carbonate alcalin à réaction alcaline, avantageusement une solution concentrée de "soude" (carbonate de sodium) au mélange réactionnel n'ayant pas subi de séparation de ses constituants, provenant de la réaction de l'épichlorohydrine avec l'α-monochlorohydrine ou à un polyglycérol supérieur.

5. Procédé selon une ou plusieurs des revendications 1 à 4, caractérisé en ce qu'on mélange avec déplacement forcé (par agitation) le mélange réactionnel, non séparé, provenant de la réaction de l'épichlorohydrine avec l' α-monochlorohydrine et qui contient du diglycérol et éventuellement des polyglycérols supérieurs, avec la solution de "soude", en ajoutant le mélange réactionnel, non soumis à séparation, provenant de la réaction de l'épichlorohydrine avec l'α-monochlorohydrine à la solution de "soude" placée préalablement.

6. Procédé selon une ou plusieurs des revendications 1 à 5, caractérisé en ce que le mélange formé par le mélange réactionnel et par la solution aqueuse à réaction alcaline présente un domaine de pH de:
7,0 à 13 , avantageusement
de 8 à 12.

7. Procédé selon une ou plusieurs des revendications 1 à 6, caractérisé en ce qu'on refroidit après la réaction le mélange réactionnel jusqu'à la température ambiante et qu'on sépare, avantageusement par filtration, la majeure partie du sel précipité.

8. Procédé selon une ou plusieurs des revendications 1 à 7, caractérisé en ce que la régénération de la masse échangeuse de cations dans les échangeurs de cations a lieu à l'aide d'une régénération effectuée par un courant se déplaçant dans le même sens ou par un courant mixte ou à plusieurs couches se déplaçant dans le même sens.

9. Procédé selon une ou plusieurs des revendications 1 à 8, caractérisé en ce que le passage de la solution contenant du diglycérol dans l'échangeur d'ions a lieu sous surpression.

10. Procédé selon une ou plusieurs des revendications 1 à 10, caractérisé en ce que l'on dirige la solution, contenant du diglycérol, sous une pression de 1,1 à 10 bars, avantageusement de 2 à 6 bars, pour lui faire traverser un ou plusieurs échangeurs de cations et au moins un échangeur d'anions.

11. Procédé selon l'une ou plusieurs des revendications 1 à 10, caractérisé en ce que la masse d'échangeurs d'ions de l'échangeur de cations et/ou de l'échangeur d'anions est recouverte d'une plaque-tamis, d'une plaque à trous ou d'un dispositif déplaçable dans le sens de la hauteur de l'échangeur d'ions recouvrant la masse de l'échangeur et permettant un passage uniforme du liquide, et/ou par une matière inerte moulée et/ou par une matière plastique élastique.

12. Procédé selon une ou plusieurs des revendications 1 à 11, caractérisé en ce que les masses d'échangeurs de cations et les masses d'échangeurs d'anions peuvent supporter la température jusqu'au-delà de 100°C.

13. Procédé selon une ou plusieurs des revendications 1 à 12, caractérisé en ce que la masse ou composition échangeuse de cations, fortement acide et la masse ou composition échangeuse d'anions, faiblement basique, présentent une surface spécifique intérieure (mesurée selon la méthode BET) de 50 à 100 m²/g.

14. Procédé selon l'une ou plusieurs des revendications 1 à 13, caractérisé en ce que la solution contenant du diglycérol est guidée dans son passage à travers les échangeurs d'ions en ayant une vitesse d'écoulement de 0,5 m/h à 15 m/h, avantageusement de 1m/h à 5m/h.
